# EUROPÄISCHE PATENTSCHRIFT

(11) **EP 3 322 387 B1**
(45) Veröffentlichungstag und Bekanntmachung des Hinweises auf die Patenterteilung: **01.05.2024**
(21) Anmeldenummer: 16740989.5
(22) Anmeldetag: 13.07.2016
(51) Int. Cl.: A61F 2/958

(54) **BALLONKATHETER**
BALLOON CATHETER
CATHÉTER À BALLONNET

(30) Priorität: 13.07.2015 DE 102015008784
(43) Veröffentlichungstag der Anmeldung: 23.05.2018
(73) Patentinhaber: JOTEC GmbH, 72379 Hechingen (DE)
(72) Erfinder: BARTHOLD, Franz-Peter, 72336 Balingen (DE)
(74) Vertreter: Witte, Weller & Partner Patentanwälte mbB
(86) Internationale Anmeldenummer: PCT/EP2016/066581
(87) Internationale Veröffentlichungsnummer: WO 2017/009356

(56) Entgegenhaltungen:
- WO-A1-00/27454
- DE-T2- 3 779 539
- US-A- 5 725 535
- US-B1- 6 527 739

## Beschreibung

Die vorliegende Erfindung betrifft einen Katheter zum Expandieren und Freisetzen einer expandierbaren endoluminalen Prothese, wobei der Katheter einen Dilatationskörper, durch dessen Dilation der Stent expandierbar ist aufweist, und ferner einen Katheterschaft mit einem proximalen ersten und einem distalen Ende; dabei weist der Katheterschaft ein Fluid-Lumen auf, über welches dem Dilatationskörper ein Fluid zuleitbar ist, und der Dilatationskörper ist am Katheterschaft angebracht, derart, dass er durch Zuleitung eines Fluids dilatierbar ist.

Derartige Katheter sind im Stand der Technik bekannt, und werden zum zum Expandieren einer expandierbaren endoluminalen Prothese, bspw. in Blutgefäßen, eingesetzt.

Der Einsatz von derartigen expandierbaren endoluminalen Prothesen, die auch als Stents oder Stentgrafts bezeichnet werden, ist im Stand der Technik bspw. bei Gefäßverengungen bekannt. Die verengten oder verschlossenen Blutgefäße werden dabei mittels Ballondilatation oder anderer Verfahren erweitert oder wiedereröffnet.

Eine ernsthaftere Blockierung der Blutgefäße von betroffenen Patienten kann zu Bluthochdruck, ischämischer Verletzung, Schlaganfall oder zu einem myokardialen Infarkt führen. Arteriosklerotische Läsionen, die den koronaren Blutfluss begrenzen oder blockieren, sind die Hauptursache der ischämischen Herzerkrankung.

Die Ballonkatheter werden fast immer von der Leiste aus über einen Führungsdraht und Führungskatheter in die Stenose platziert und mit Druck aufgeblasen. Dadurch wird die Engstelle beseitigt und eine Operation vermieden. Zusätzlich werden dabei, wie oben erwähnt, häufig Stents (Drahtgeflechte, die das Gefäß von innen schienen und offen halten sollen) oder Stentgrafts (Drahtgeflechte mit Ummantelung) implantiert.

Stents und Stentgrafts werden aber auch bspw. in der Behandlung von Aneurysmen eingesetzt. Dabei versteht man unter einem Aneurysma eine Ausweitung oder Aussackung eines Blutgefäßes infolge angeborener oder erworbener Wandveränderungen. Die Aussackung kann dabei die Gefäßwand als Ganzes erfassen oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysmas kann im fortgeschrittenen Stadium zu einer Ruptur des Blutgefäßes führen, mit der Folge, dass der Patient innerlich verblutet.

Die zur Behandlung von Gefäßverengungen verwendeten Stents/Stentgrafts bzw. Gefäßprothesen bestehen dabei im Allgemeinen aus einem röhrchenförmigen/hohlzylindrischen Metallrahmen, oder aus einzelnen, hintereinander angeordneten Metall-(Stent)federn, dessen bzw. deren Mantelfläche im Falle von Stentgrafts mit einer Textil- oder Polymerfolie abgedeckt ist, so dass sich ein hohlzylindrischer Körper ergibt. Der Stent/Stentgraft wird hierzu in der Regel im gekrimpten Zustand auf einen Ballonkatheter geladen und mittels eines Führungsdrahts in den Bereich der Läsion gebracht, wo der Stent freigesetzt werden soll. Hierzu wird der Ballon und mit diesem der Stent bzw. Stentgraft aufgedehnt und gegen die Gefäßwand gedrückt. Dabei wird der Stent/Stentgraft plastisch verformt und verbleibt in diesem aufgedehnten Zustand mit größerem Durchmesser als im geladenen Zustand auf dem Ballonkatheter. Dadurch wird auch die Läsion offen gehalten, und das Therapieziel erreicht. Der Ballonkatheter wird nach der Expansion des Stents/Stentgrafts wieder deflatiert und aus dem Gefäß entfernt.

Die im Stand der Technik bekannten Ballonkatheter werden bei Dilatation eines Stents/Stentgrafts zum Teil einem hohen hydraulischen Druck von bis zu 30 bar ausgesetzt. Dies wird technisch durch sogenannte "non-compliant- Ballons" erreicht, also Konstrukten aus extrem stabilen Folienschläuchen, die solchen Drücken widerstehen und sich dabei nicht weiter als bis zu ihrem Nenndurchmesser aufdehnen lassen. Dies ist ein wichtiger Sicherheitsaspekt, um eine Überdehnung des Gefäßes zu verhindern.

der Nachteil einer solchen Konstruktion besteht darin, dass der Ballonkatheter im dilatierten Zustand durch seinen hohen Innendruck immer gerade sein muss. Die Gefäßanatomie erfordert jedoch oftmals eine gekrümmte oder flexible Anordnung. Dies wirkt sich vor allem bei längeren Läsionen sehr nachteilig aus, da das Gefäß in die gerade Form des Stents gezwungen wird und dort auch verbleibt.

Aufgabe der vorliegenden Erfindung ist es daher, ein System bereitzustellen, mit dem die oben beschriebenen Nachteile überwunden werden können. Die DE 3779539 beschreibt Gefäßkatheter mit unabhängig voneinander aufblasbaren/entleerbaren Ballons.

Erfindungsgemäß wird diese Aufgabe durch einen Katheter zum Expandieren und Freisetzen einer expandierbaren endoluminalen Prothese gelöst, wobei der Katheter folgendes aufweist: einen Dilatationskörper, durch dessen Dilation eine expandierbare Prothese expandierbar ist, einen Katheterschaft mit einem proximalen ersten und einem distalen Ende, wobei der Katheterschaft ein Fluid-Lumen aufweist, über welches dem Dilatationskörper ein Fluid zuleitbar ist; ferner ist der Dilatationskörper am Katheterschaft angebracht, derart, dass er durch Zuleitung eines Fluids dilatierbar ist; der erfindungsgemäße Katheter zeichnet sich dadurch aus, dass der Dilatationskörper aus zumindest drei Ballonsegmenten ausgebildet ist, die auf dem Katheterschaft aneinander unmittelbar angrenzend und hintereinander liegend angeordnet und unabhängig voneinander dilatierbar sind.

Die der Erfindung zugrunde liegende Aufgabe wird auf diese Weise vollkommen gelöst.

Mit dem erfindungsgemäßen Katheter, dessen Gesamtlänge mehrere, also mindestens drei Segmente mit einzeln befüll- und expandierbaren bzw. dilatierbaren Ballons aufweist, bzw. hieraus besteht, ist es möglich, dass der einzusetzende expandierbare Stent/Stentgraft auch in einem gekrümmten Gefäß aufgeweitet werden kann, ohne dieses traumatisch zu verändern. Dies wird durch die Segmentierung des Dilatationskörpers des Katheters erreicht, sowie dadurch, dass diese einzeln und sequentiell ansteuerbar sind.

Die Ballonsegmente sind dabei definitionsgemäß unmittelbar hintereinander angrenzend nacheinander angeordnet und bilden damit einzelne bzw. einzeln inflatierbare/dilatierbare und einzeln ansteuerbare Segmente, die ggf. über ihre jeweiligen Enden miteinander in Berührung stehen können.

Gemäß einer Ausführungsform des erfindungsgemäßen Katheters weist dieser entsprechend ein Fluid-Lumen auf, über welche den Ballonsegmenten sequentiell ein Fluid zuleitbar ist. Bei dieser Ausführungsform wird den Ballonsegmenten also über ein Fluid-Lumen ein Fluid zugeleitet, wobei über entsprechend vorgesehene jeweilige Dichtungen und/oder Ventile sicher gestellt ist, dass die Ballonsegmente sequentiell angesteuert und dilatiert werden können. Dabei kann bspw. vorgesehen sein, dass jeweils einem Ballonsegment eine vorbestimmte Menge an Fluid zugeleitet wird, bevor dem sequentiell unmittelbar nachgeordneten Ballonsegment eine ggf. definierte Menge Fluid zugeleitet wird.

Gemäß einer weiteren Ausführungsform des Katheters weist dieser ein Fluid-Lumen für jedes der Ballonsegmente auf, über welche den Ballonsegmenten jeweils ein Fluid zuleitbar ist. Bei dieser Ausführungsform ist also jedem Ballonsegment ein Fluid-Lumen zugeordnet, über welche die Ballonsegmente einzeln und sequentiell ansteuerbar sind.

Vorliegend kann das Fluid ein Gas oder eine Flüssigkeit, bspw. Saline oder ein röntgendichtes Kontrastmedium, sein, und jedes in diesem Bereich üblicherweise zur Dilatation von Dilatationskörpern eingesetzte Fluid, wobei dem Fachmann hierzu hinreichende Möglichkeiten bekannt sein werden. Dabei wird er berücksichtigen, dass eine Dilatation und die Deflation mit Gas schneller bewirkt werden kann als mit einer Flüssigkeit. Das Fluid-Lumen kann dabei durch den vollständigen Katheterschaft geführt sein, oder aber nur im Bereich der Ballonsegmente. Das Fluid-Lumen steht mit Öffnungen bzw. Zuleitungen mit den Ballonsegmenten in Kontakt, wobei jeweils ein Ballonsegment über eine Öffnung verfügt, über welche das Fluid zugeleitet wird. Unter "Öffnung" wird dabei jede Fluid-Verbindung zwischen dem Inneren eines Ballonsegments, bspw. eine Bohrung, Öffnung oder ein Zugang, und dem Lumen des Fluid-Lumens verstanden.

Gemäß einer weiteren Ausführungsform weisen die Ballonsegmente jeweils zwei Endabschnitte auf, über welche sie am Katheterschaft fixiert sind, und jeweils einen mittleren Abschnitt, der sich zwischen den beiden Endabschnitten erstreckt, der nicht am Katheterschaft fixiert ist, und der durch Zuleitung eines Fluids dilatierbar ist.

Erfindungsgemäß ist bevorzugt, wenn mindestens drei, vorzugsweise zwischen 3 und 15, insbesondere 4, 5, 6, 7, 8, 9, 10, 11, 12, 13, 14, oder 15 Ballonsegmente vorgesehen sind, denen über eines oder mehrere Fluid-Lumen ein Fluid zuleitbar ist.

Die konkrete Anzahl der Ballonsegmente wird der Fachmann anhand der vorliegenden Lehre und unter Berücksichtigung des einzusetzenden Stents/ Stentgrafts sowie der zu berücksichtigenden jeweiligen Gefäßanatomie eines Patienten bestimmen können.

Ferner ist bei dem erfindungsgemäßen Katheter bevorzugt, wenn ein einzelnes Ballonsegment eine Länge von wenigstens 3 mm, insbesondere wenigstens 5 oder 7 mm, und vorzugsweise wenigstens 10 mm, und maximal 20 mm aufweist.

Der Durchmesser des Ballons liegt dabei vorzugsweise zwischen 5 und 20 mm.

Auch bezüglich der Längen der Ballonsegmente wird der Fachmann anhand der vorliegenden Lehre und unter Berücksichtigung der Anzahl der Ballonsegmente, für den jeweiligen Patienten bzw. für den jeweils zu behandelnden Gefäßabschnitt geeignete Größen und Dimensionen auffinden können.

Gemäß einer Ausführungsform der vorliegenden Erfindung weist der Katheterschaft dabei weiterhin ein Führungsdraht-Lumen auf, das unterschiedlich von den Fluid-Lumen ist.

Gemäß einer weiteren Ausführungsform des erfindungsgemäßen Katheters weisen die Ballonsegmente jeweils eine Wandung auf, deren Wandstärke jeweils einheitlich oder uneinheitlich über die Abschnitte eines einzelnen Ballonsegments ist. Vorteilhafterweise kann bei dieser Ausführungsform bspw. berücksichtigt werden, dass ggf. die Wandstärke des Ballonsegmentabschnitts, der zum jeweils benachbarten Ballon zu liegen kommt, anders, also kleiner oder größer, ausgestaltet sein kann als die Wandstärke der Ballonsegmentabschnitte, die gegen die Gefäßwand drücken, oder aber die Wandstärke eines Ballonsegment ist über seine gesamte Länge und in allen seinen jeweiligen Abschnitten gleich. Dabei können unterschiedliche Ballonsegmente auch unterschiedliche oder gleiche Wandstärken jeweils über ihre Abschnitte hinweg aufweisen.

Gemäß einer weiteren Ausführungsform des erfindungsgemäßen Katheters weisen die Ballonsegmente jeweils eine Wandung mit einer Wandstärke auf, wobei die Wandstärken der Ballonsegmente unterschiedlich oder einheitlich sind. Bei dieser Ausführungsform kann also vorteilhafterweise berücksichtigt werden, wenn die Ballonsegmente, die hintereinander über die Länge des Katheters hinweg angeordnet sind, unterschiedliche Wandstärken im Vergleich untereinander aufweisen, so dass auch hier ggf. eine Anpassung an unterschiedliche Gefäßanatomien berücksichtigt werden können.

Auch solche Ballonsegmente, die unterschiedliche Wandstärken aufweisen, können wiederum auf sich bezogen auf ihren jeweiligen Abschnitten unterschiedliche Wandstärken aufweisen.

In einer Weiterbildung des erfindungsgemäßen Katheters ist bevorzugt, wenn die äußere Oberfläche der Ballonsegmente mit einer Substanz beschichtet ist, die ausgewählt ist aus: einem Medikament, einer hydrophilen Beschichtung, einer Heparinbeschichtung oder einer Kombination hieraus.

Beschichtete Ballonkatheter sind im Stand der Technik bekannt, und grundsätzlich kommt jeder pharmazeutische Wirkstoff, bspw. Rapamycin, Paclitaxel, o.ä., der die Offenhaltung des betroffenen Gefäßes unterstützt oder fördert, als Beschichtung in Betracht.

Der vorliegend offenbarte Katheter kann in der Angioplastie, d.h. zur Behandlung von Stenosen verwendet werden, wobei die Behandlung ohne die Freisetzung eines Stents/Stentgrafts erfolgt. Dabei ist der Dilatationskörper derart ausgebildet, dass er zum Dilatieren einer Stenose in einem Blutgefäß geeignet ist.

Erfindungsgemäß sind die Dilatationssegmente Ballonsegmente.

Unter "Ballon" wird dabei vorliegend jede elastische Hülle verstanden, die - zumindest im gefüllten Zustand - selbsttragend und gas-/flüssigkeitsdicht ist und die eine im Wesentlichen bauchige Form aufweist, die mit einem Fluid, also einem Gas oder einer Flüssigkeit, gefüllt werden kann. Die Hülle kann dabei eine symmetrische oder asymmetrische Form aufweisen.

Gemäß einer Ausführungsform ist bevorzugt, wenn die Ballonsegmente im dilatierten Zustand jeweils eine Form aufweisen, die ausgewählt ist aus im Wesentlichen rund oder eiförmig. Die Form der Ballonsegmente wird dabei in der Regel durch die jeweiligen Materialien, aus denen die Ballonsegemente gebildet werden, vorgegeben, wobei die Ballonsegmente entsprechend aus einem sogenannten "non-compliant", d.h. nicht über eine bestimmte Endabmessung dilatierbaren/dehnbaren Material sind.

Gemäß einer weiteren Ausführungsform ist bevorzugt, wenn die Form der Ballonsegmente länglich ist, mit einem ersten und einem zweiten Ende, wobei "länglich" jede Form umfasst, die in axialer Richtung länger ist als in radialer Richtung. Bei dieser Ausführungsform kann bspw. das proximale Ende eines Ballonsegements nach außen gewölbt sein, und das distale Ende nach innen gewölbt, wobei ein nach außen gewölbtes proximales Ende eines hinter einem ersten Ballonsegment angeordneten zweiten Ballonsegments in die nach innen gerichtete Wölbung des ersten Ballonsegments eingreift. Dabei beziehen sich "nach innen" und "nach außen" immer auf den einzelnen Körper eines Ballonsegments. Gemäß einer weiteren Ausführungsform weist bspw. das am weitesten distal gelegene Ballonsegment an seinen beiden Ende eine nach außen gerichtete Wölbung auf, da in dessen zweites Ende kein weiteres Ballonsegment eingreift.

Unter einem "Dilatationskörper" wird vorliegend jedes elastische Element verstanden, das sich unter Ausübung von Druck, bspw. durch Zufuhr eines Fluids, im Hinblick auf sein Volumen vergrößern bzw. expandieren kann.

Gemäß einer Weiterbildung des erfindungsgemäßen Katheters ist im Fluid-Lumen ein Dichtungselement vorgesehen, welches die einzelnen Dilatationssegmente ansteuert.

Dabei ist gemäß einer weiteren Ausführungsform vorgesehen, wenn das Dichtungselement im Fluid-Lumen lateral steuerbar ausgeführt ist.

Hierin beschrieben ist auch die Verwendung eines hier offenbarten Katheters zur Freisetzung einer expandierbaren endoluminalen Prothese in einem Gefäß eines Patienten zur Behandlung einer Gefäßverengung.

Vorliegend beschrieben ist ferner ein Verfahren zur Freisetzung einer expandierbaren endoluminalen Prothese in einem Gefäß eines Patienten, wobei das Verfahren die folgenden Schritte aufweist:
a) Bereitstellen einer auf einen erfindungsgemäßen Katheter aufgebrachten expandierbaren endoluminalen Prothese;
b) Einführen des mit der expandierbaren endoluminalen Prothese beladenen Katheters bis zu der zu behandelnden Stelle in dem Gefäß;
c) sequentielles Dilatieren der Ballonsegmente zur Expansion und Freisetzung der expandierbaren endoluminalen Prothese in dem Gefäß.

Die expandierbare endoluminale Prothese kann dabei an einer eine Krümmung aufweisenden Stelle im Gefäß freigesetzt werden.

Das Gefäß kann dabei ein Blutgefäß oder ein anderes Hohlorgan eines bspw. menschlichen Patienten sein, wie bspw. auch der Gallengang oder die Harnröhre.

Vorliegend offenbart ist auch ein Verfahren zur Behandlung einer Stenose in einem Gefäß, bspw. eines Blutgefäßes oder eines anderen Hohlorgan eines Patienten, bspw. eines Menschen, wobei das Verfahren die folgenden Schritte aufweist:
a) Bereitstellen eines erfindungsgemäßen Katheters, der ggf. beladen ist mit einer expandierbaren endoluminalen Prothese;
b) Einführen des Katheters aus Schritt a) bis zu der zu behandelnden Stelle in dem Gefäß;
c) sequentielles Dilatieren der Ballonsegmente, sowie ggf. dadurch Expansion und Freisetzung der expandierbaren endoluminalen Prothese in dem Gefäß, zur Behandlung der Stenose, derart dass das Gefäß erweitert oder wiedergeöffnet wird.

Das Material, aus dem die Ballonsegmente des erfindungsgemäßen Katheters dabei bestehen oder ein solches aufweisen, ist vorzugsweise ein Polymer, und bspw. ausgewählt aus der Gruppe umfassend Polyurethan, Polyether-Polyurethan, Polyethylenterephthalat, Polybutylenterephthalat, Polyamid sowie Copolymere und Mischungen davon.

In den Ausführungsformen, in denen der Ballon beschichtet ist, kann bspw. der pharmazeutische Wirkstoff bereits in dem Polymer, aus dem die Ballonsegmente gebildet werden, enthalten sein. Alternativ kann der pharmazeutische Wirkstoff auch auf das getrocknete und/oder gereinigte Ballonsegment aufgetragen werden, wobei die Auftragung hier vorzugsweise im expandierten Zustand des Ballonsegments erfolgen sollte. Der Wirkstoff beziehungsweise die Wirkstoffzusammensetzung besitzt idealerweise nur eine minimale Wechselwirkungen mit der Polymermembran des Ballonsegments, um die Freisetzung des Wirkstoffs zu erleichtern.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in Bezug auf diese nachstehend näher beschrieben. Es zeigen:
- Fig. 1: eine schematische Darstellung einer ersten Ausführungsform eines erfindungsgemäßen Katheters, vor dem Start der Dilation der Ballonsegmente (ohne Prothesenbeladung);
- Fig. 2: die in Fig. 1 gezeigte schematische Darstellung der ersten Ausführungsform, wobei hier das proximal gelegene Ballonsegment im dilatierten Zustand gezeigt ist;
- Fig. 3: die in Fig. 1 und 2 gezeigte schematische Darstellung der ersten Ausführungsform in einem Zustand, in dem das zwischen dem proximal und distal gelegenen Ballonsegment in einem dilatierten Zustand gezeigt ist;
- Fig. 4: die in den Figuren 1 bis 3 gezeigte schematische Darstellung der ersten Ausführungsform in einem Zustand in den alle Ballonsegmente dilatiert sind; und
- Fig. 5: eine schematische Darstellung einer weiteren Ausführungsform eines erfindungsgemäßen Katheters (A); sowie eine schematische Detailansicht eines Ballonsegments (B).

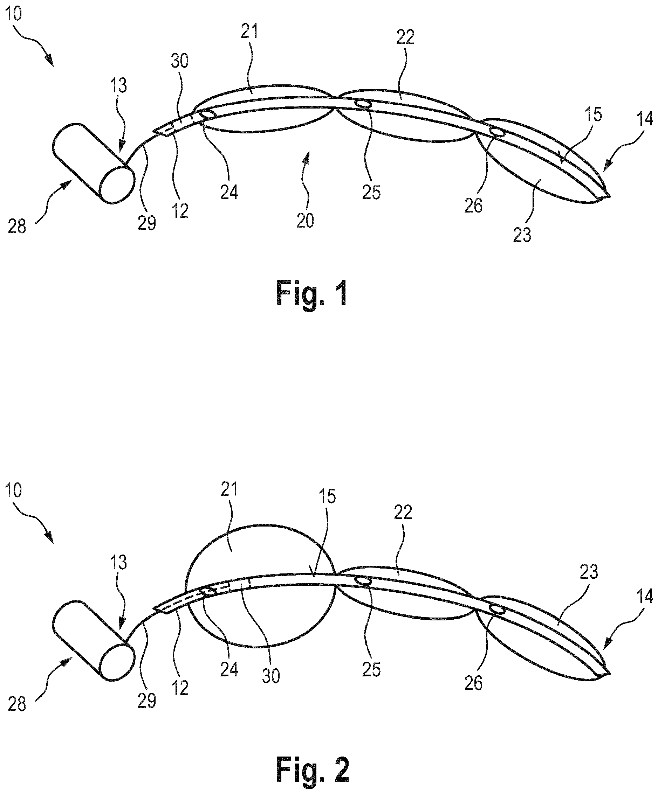

In den Figuren 1 bis 4 ist mit 10 eine beispielhafte Ausführungsform eines erfindungsgemäßen Katheters gezeigt, mit einem Katheterschaft 12 mit einem proximalen ersten Ende 13 und einem distalen Ende 14, wobei der Katheterschaft 12 ein Fluid-Lumen 15 aufweist.

Der erfindungsgemäße Katheter 10 in Fig. 1 weist ferner einen Dilatationskörper 20 auf, der in dem in Fig. 1 gezeigten Beispiel aus 3 getrennten, aneinander unmittelbar angrenzenden und hintereinander liegend angeordneten Ballonsegmenten 21, 22, 23 besteht, die unabhängig voneinander dilatierbar sind. Die Form der in den Figuren 1 bis 4 gezeigten Ballonsegmente ist im Wesentlichen rund bzw. eiförmig. Es versteht sich, dass der erfindungsgemäße Katheter auch mehr als drei Ballonsegmente aufweisen kann.

Wie in den Figuren 1 bis 4 gezeigt, weisen die einzelnen Ballonsegmente eine "im Wesentlichen" runde bzw. eiförmige Form dahingehend auf, dass sie im expandierten Zustand nicht eine identische Form aufweisen müssen, jedoch eine Form, die die wesentlichen Merkmale der jeweils anderen Formen aufweist.

Im Fluid-Lumen 15 sind Öffnungen 24, 25, 26 vorgesehen, über welche jeweils Fluid den Ballonsegmenten 21, 22 und 23 zugeführt werden kann.

Der in den Figuren 1 bis 4 gezeigte erfindungsgemäße Katheter 10 weist ferner an seinem proximalen Ende 13 eine Steuerung 28 auf, über welche die Zuleitung des Fluids zu den einzelnen Ballonsegmenten 21, 22 und 23 gesteuert werden kann.

Gemäß der in den Fig. 1 bis 3 gezeigten Ausführungsform, die in den Figuren 1 bis 4 in unterschiedlichen dilatierten Zuständen der Ballonsegmente gezeigt ist, umfasst die Steuerung 28 ein Steuerkabel 29 sowie ein Dichtelement 30, mit Hilfe derer die Zuleitungen zu den einzelnen Ballonsegmenten abgedichtet werden kann, sobald der gewünschte Dilatationszustand eines jeweiligen Ballonsegments erreicht ist.

Wie den Fig. 1 bis 4 zu entnehmen ist, kann durch Bewegen des Dichtelements 30 in distale Richtung, also vom Anwender weg, die Zuleitung des die einzelnen Ballonsegmente dilatierende Fluids gesteuert werden, indem das Dichtelement 30 die Zuleitung bzw. Öffnungen, 24, 25, und 26 der jeweiligen Ballonsegmente 21, 22 und 23 abdichtet, sobald die Dichtung diese Öffnungen 24, 25 und 26 in distale Richtung passiert.

Dadurch kann ein sequentielles Befüllen und Dilatieren der Ballonsegmente 21, 22 und 23 erreicht werden.

Alternativ kann die Steuerung auch derart erfolgen, dass die Zuleitungen bzw. Öffnungen 24, 25, 26 zu den jeweiligen Ballonsegmenten 21, 22 und 23 erst dann geöffnet werden, wenn das Dichtelement 30 diese jeweils passiert.

Aufgrund ihrer jeweiligen Ausbildung als "non-compliance" Ballonsegmente 21, 22, 23 können sich diese nur bis zu einer räumlich vorbestimmten Ausdehnung dilatieren.

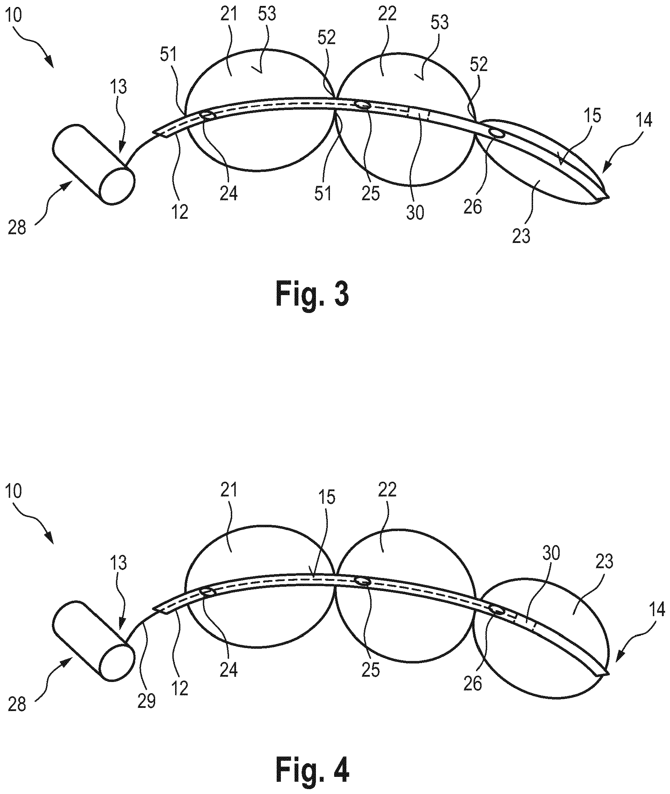

Mit beispielhaftem Verweis auf Fig. 3 ist ferner zu sehen, dass jedes der Ballonsegmente 21, 22, 23 jeweils zwei Endabschnitte 51, 52 aufweist, über welche die Ballonsegmente 21, 22, 23 am Katheterschaft fixiert sind, und einen mittleren Abschnitt 53, der nicht am Katheterschaft fixiert ist, und der durch Zuleitung eines Fluids dilatierbar ist.

Die Steuerung kann dabei von proximal nach distal oder aber von distal nach proximal erfolgen, ebenso wie die Dilatation der einzelnen Ballonsegmente 21, 22 und 23, die entsprechend in der Reihenfolge von proximal nach distal oder aber von distal nach proximal sequentiell dilatiert werden können.

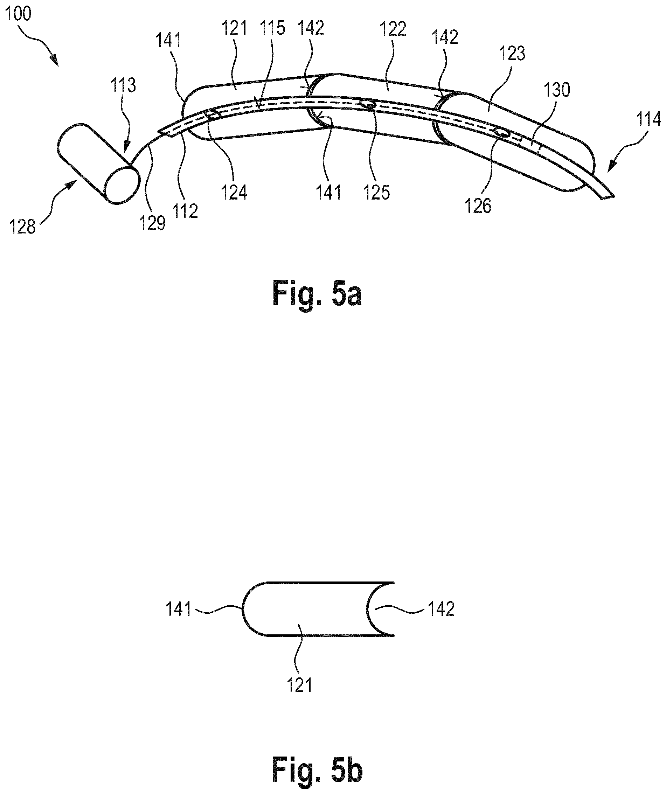

Fig. 5 zeigt schließlich eine weitere Ausführungsform eines erfindungsgemäßen Katheters 100, mit einem Katheterschaft 112, mit einem proximalen ersten Ende 113 und einem distalen Ende 114, wobei der Katheterschaft 112 ein Fluid-Lumen 115 aufweist.

Der erfindungsgemäße Katheter 110 in Fig. 5 weist ferner einen Dilatationskörper 120 auf, der in dem in Fig. 1 gezeigten Beispiel aus 3 getrennten, aneinander unmittelbar angrenzenden und hintereinander liegend angeordneten Ballonsegmenten 121, 122, 123 besteht, die unabhängig voneinander dilatierbar sind. Die Form eines Ballonsegments 121, 122, und 123 ist in der in Fig. 5 gezeigten Ausführungsform länglich, und zwar derart, dass es jeweils ein erstes Ende 141 und ein zweites Ende 142 aufweist. Das erste Ende 141 ist dabei, in Bezug auf das jeweilige Ballonsegment 121, 122, 123, im expandierten Zustand nach außen gewölbt, und das zweite Ende 142 ist, ebenfalls in Bezug auf das gleiche jeweilige Ballonsegment 121, 122, 123 im expandierten Zustand nach innen gewölbt. Dabei greift ein nach außen gewölbtes erstes Ende 141 eines hinter einem ersten Ballonsegment 121 angeordneten zweiten Ballonsegments 122 in die nach innen gerichtete Wölbung des ersten Ballonsegments 121 ein.

Im Fluid-Lumen 115 sind Öffnungen 124, 125, 126 vorgesehen, über welche jeweils den Ballonsegmenten 121,1 22 und 123 ein Fluid zugeführt werden kann.

Der in Fig. 5A gezeigte erfindungsgemäße Katheter 110 weist ferner an seinem proximalen Ende 113 eine Steuerung 128 auf, über welche die Zuleitung des Fluids zu den einzelnen Ballonsegmenten 121,122 und 123 gesteuert werden kann.

Gemäß der in Fig. 5A gezeigten Ausführungsform umfasst die Steuerung 28 ebenfalls ein Steuerkabel 29 sowie ein Dichtelement 130, mit Hilfe derer die Zuleitungen bzw. Öffnungen 124, 125 und 126 zu den einzelnen Ballonsegmenten 121, 122 und 123 ansteuerbar sind und bspw. abgedichtet werden können, sobald der gewünschte Dilatationszustand eines jeweiligen Ballonsegments 121, 122, 123 erreicht ist.

Wie Fig. 5A ferner zu entnehmen ist, hat das Ballonsegment 123, das am weitesten distal angeordnet ist, eine längliche Ballonform, mit einem ersten und einem zweiten Ende, die jeweils nach außen gewölbt sind. Dieses Element ist in Fig. 5B im Detail genauer gezeigt.

Für den Einsatz der erfindungsgemäßen Ausführungsbeispiele des Katheters 10, 100 wird dieser entweder mit einer expandierbaren Prothese, bspw. einem Stent/Stentgraft (nicht gezeigt) beladen, wobei der expandierbare Stent/Stentgraft in der Regel aufgekrimpt wird, und zu der gewünschten Stelle im Gefäß vorgeschoben, vorzugsweise über einen Führungsdraht. Sobald der Stent/Stentgraft an der gewünschten Stelle, vorzugsweise in einer Krümmung eines Gefäßes, positioniert ist, werden die Ballonsegmente 21, 22, 23, oder 121, 122, 123 sequentiell durch Zuleitung eines Fluids nacheinander dilatiert, und zwar in der Reihenfolge, wie sie hintereinander angeordnet sind. Dadurch drücken sie den expandierbaren Stent/Stentgraft radial nach außen und gegen die Gefäßwand, wodruch diese abgestützt oder wieder geöffnet wird.

Sobald der Stent/Stentgraft expandiert ist, werden die Ballonsegmente wieder deflatiert und der Katheter 10, 100 aus dem Gefäß entfernt, wohingegen der Stent/Stentrgaft im Gefäß verbleibt.

## Patentansprüche

1. Katheter (10; 100) zum Expandieren einer expandierbaren endoluminalen Prothese, wobei der Katheter (10; 100) folgendes aufweist:
- einen Dilatationskörper (20), durch dessen Dilation eine auf dem Katheter (10; 100) ggf. geladene expandierbare Prothese expandierbar ist,
- einen Katheterschaft (12) mit einem proximalen ersten (13) und einem distalen (14) Ende, wobei der Katheterschaft (12) ein Fluid-Lumen (15) aufweist, über welches dem Dilatationskörper (20) ein Fluid zuleitbar ist,
- wobei der Dilatationskörper (20) am Katheterschaft (12) angebracht ist, derart, dass er durch Zuleitung eines Fluids dilatierbar ist,
wobei der Dilatationskörper (20) aus zumindest drei getrennten Ballonsegmenten (21, 22, 23; 121, 122, 123) ausgebildet ist, die auf dem Katheterschaft (12) aneinander unmittelbar angrenzend und hintereinander liegend angeordnet und unabhängig voneinander dilatierbar sind, **dadurch gekennzeichnet, dass** der Katheter (10; 100) ein einziges Fluid-Lumen (15) aufweist, über welche den Ballonsegmenten (21, 22, 23; 121, 122, 123) sequentiell ein Fluid zuleitbar ist.

2. Katheter (10; 100) nach Anspruch 1, **dadurch gekennzeichnet, dass** die Ballonsegmente (21, 22, 23; 121, 122, 123) jeweils zwei Endabschnitte aufweisen, über welche sie am Katheterschaft fixiert sind, und jeweils einen mittleren Abschnitt, der sich zwischen den beiden Endabschnitten erstreckt, der nicht am Katheterschaft (12) fixiert ist, und der durch Zuleitung eines Fluids dilatierbar ist.

3. Katheter (10; 100) nach einem der vorstehenden Ansprüche, **dadurch gekennzeichnet, dass** zwischen 3 und 15 Ballonsegmente (21, 22, 23; 121, 122, 123) vorgesehen sind, denen über ein einziges Fluid-Lumen (15) ein Fluid zuleitbar ist.

4. Katheter (10; 100) nach einem der vorstehenden Ansprüche, **dadurch gekennzeichnet, dass** ein einzelnes Ballonsegment (21, 22, 23; 121, 122, 123) eine Länge von wenigstens 3 mm, insbesondere wenigstens ab 5 mm, und vorzugsweise wenigstens 10 mm aufweist.

5. Katheter (10; 100) nach einem der vorstehenden Ansprüche, **dadurch gekennzeichnet, dass** der Katheterschaft (12)ein Führungsdraht-Lumen aufweist, das unterschiedlich von den Fluid-Lumen (15) ist.

6. Katheter (10; 100) nach einem der vorstehenden Ansprüche, **dadurch gekennzeichnet, dass** die Ballonsegmente(21, 22, 23; 121, 122, 123) jeweils eine Wandung aufweisen, deren Wandstärke jeweils einheitlich oder uneinheitlich über die Abschnitte eines einzelnen Ballonsegments (21, 22, 23; 121, 122, 123) ist.

7. Katheter (10; 100) nach einem der vorstehenden Ansprüche, **dadurch gekennzeichnet, dass** die Ballonsegmente (21, 22, 23; 121, 122, 123) jeweils eine Wandung mit einer Wandstärke aufweisen, wobei die Wandstärken der Ballonsegmente (21, 22, 23; 121, 122, 123) unterschiedlich oder einheitlich sind.

8. Katheter (10; 100) nach einem der vorstehenden Ansprüche, **dadurch gekennzeichnet, dass** die äußere Oberfläche der Ballonsegmente (21, 22, 23; 121, 122, 123) mit einer Substanz beschichtet ist, die ausgewählt ist aus: einem pharmazeutischen Wirkstoff, einer hydrophilen Beschichtung, einer Heparinbeschichtung oder einer Kombination hieraus.

9. Katheter (10; 100) nach einem der vorstehenden Ansprüche, **dadurch gekennzeichnet, dass** im Fluid-Lumen (15) ein Dichtungselement (30) vorgesehen ist, welches die einzelnen Ballonsegmente (21, 22, 23; 121, 122, 123) ansteuert.

10. Katheter (10; 100) nach Anspruch 9, **dadurch gekennzeichnet, dass** das Dichtungselement (30) im Fluid-Lumen (15) lateral steuerbar ausgeführt ist.

11. Katheter (10; 100) nach einem der vorstehenden Ansprüche, **dadurch gekennzeichnet, dass** er ferner eine hierauf geladene expandierbare endoluminale Prothese aufweist.

## Claims

1. A catheter (10; 100) for expanding an expandable endoluminal prosthesis, said catheter (10; 100) comprising the following:
- a dilation body (20) whose dilation permits the expansion of an expandable prosthesis optionally loaded on the catheter (10; 100),
- a catheter shaft (12) having a proximal first end (13) and a distal end (14), said catheter shaft (12) having a fluid lumen (15) via which a fluid can be delivered to the dilation body (20),
- the dilation body (20) being mounted on the catheter shaft (12), such, that it is dilatable by delivery of a fluid,
wherein the dilation body (20) is formed by at least three separate balloon segments (21, 22, 23; 121, 122, 123) which are arranged immediately adjacent to one another and one behind the other on the catheter shaft (12) and are dilatable independently of one another, **characterized in that** the catheter (10; 100) comprises a single fluid lumen (15) via which a fluid can be delivered in sequence to the balloon segments (21, 22, 23; 121, 122, 123).

2. The catheter (10; 100) as claimed in claim 1, **characterized in that** the balloon segments (21, 22, 23; 121; 122, 123) each have two end portions via which they are fixed on the catheter shaft, and they each have a central portion which extends between the two end portions, which central portion is not fixed on the catheter shaft (12) and which is dilatable by delivery of a fluid.

3. The catheter (10; 100) as claimed in one of the preceding claims, **characterized in that** between 3 and 15 balloon segments (21, 22, 23; 121, 122, 123) are provided, to which a fluid can be delivered via a single fluid lumen (15).

4. The catheter (10; 100) as claimed in one of the preceding claims, **characterized in that** an individual balloon segment (21, 22, 23; 121, 122, 123) has a length of at least 3 mm, in particular at least 5 mm and preferably at least 10 mm.

5. The catheter (10; 100) as claimed in one of the preceding claims, **characterized in that** the catheter shaft (12) has a guidewire lumen, which is different than the fluid lumens (15).

6. The catheter (10; 100) as claimed in one of the preceding claims, **characterized in that** the balloon segments (21, 22, 23; 121, 122, 123) each comprise a wall, of which the wall thickness is in each case uniform or non-uniform over the portions of an individual balloon segment (21, 22, 23; 121, 122, 123).

7. The catheter (10; 100) as claimed in one of the preceding claims, **characterized in that** the balloon segments (21, 22, 23; 121, 122, 123) each have a wall with a wall thickness, the wall thicknesses of the balloon segments (21, 22, 23; 121, 122, 123) being different or uniform.

8. The catheter (10; 100) as claimed in one of the preceding claims, **characterized in that** the outer surface of the balloon segments (21, 22, 23; 121, 122, 123) is coated with a substance chosen from: a pharmaceutical active substance, a hydrophilic coating, a heparin coating or a combination thereof.

9. The catheter (10; 100) as claimed in one of the preceding claims, **characterized in that** a sealing element (30) is provided in the fluid lumen (15) and controls the individual balloon segments (21, 22, 23; 121, 122, 123).

10. The catheter (10; 100) as claimed in claim 9, **characterized in that** the sealing element (30) is designed to be controllable laterally in the fluid lumen (15).

11. The catheter (10; 100) as claimed in one of the preceding claims, **characterized in that** it moreover has an expandable endoluminal prosthesis loaded thereon.

## Revendications

1. Cathéter (10 ; 100) pour l'expansion d'une prothèse endoluminale expansible, le cathéter (10 ; 100) présentant les éléments suivants :
- un corps de dilatation (20), par la dilatation duquel une prothèse expansible éventuellement chargée sur le cathéter (10 ; 100) peut être expansée,
- une tige de cathéter (12) avec une première extrémité proximale (13) et une extrémité distale (14), la tige de cathéter (12) présentant une lumière de fluide (15) par le biais de laquelle un fluide peut être amené au corps de dilatation (20),
- le corps de dilatation (20) étant monté sur la tige de cathéter (12) de telle sorte qu'il puisse être dilaté par l'amenée d'un fluide,
le corps de dilatation (20) étant réalisé à partir d'au moins trois segments de ballonnet séparés (21, 22, 23 ; 121, 122, 123) qui sont agencés sur la tige de cathéter (12) de manière directement adjacente les uns aux autres et les uns derrière les autres et qui peuvent être dilatés indépendamment les uns des autres, **caractérisé en ce que** le cathéter (10 ; 100) présente une seule lumière de fluide (15) par le biais de laquelle un fluide peut être amené séquentiellement aux segments de ballonnet (21, 22, 23 ; 121, 122, 123).

2. Cathéter (10 ; 100) selon la revendication 1, **caractérisé en ce que** les segments de ballonnet (21, 22, 23 ; 121, 122, 123) présentent chacun deux sections d'extrémité par le biais desquelles ils sont fixés à la tige de cathéter, et chacun une section médiane qui s'étend entre les deux sections d'extrémité, qui n'est pas fixée à la tige de cathéter (12), et qui peut être dilatée par l'amenée d'un fluide.

3. Cathéter (10 ; 100) selon l'une quelconque des revendications précédentes, **caractérisé en ce qu'**il est prévu entre 3 et 15 segments de ballonnet (21, 22, 23 ; 121, 122, 123) auxquels un fluide peut être amené par le biais d'une seule lumière de fluide (15).

4. Cathéter (10 ; 100) selon l'une quelconque des revendications précédentes, **caractérisé en ce qu'**un segment de ballonnet individuel (21, 22, 23 ; 121, 122, 123) présente une longueur d'au moins 3 mm, en particulier d'au moins 5 mm, et de préférence d'au moins 10 mm.

5. Cathéter (10 ; 100) selon l'une quelconque des revendications précédentes, **caractérisé en ce que** la tige de cathéter (12) présente une lumière de fil de guidage qui est différente de la lumière de fluide (15).

6. Cathéter (10 ; 100) selon l'une quelconque des revendications précédentes, **caractérisé en ce que** les segments de ballonnet (21, 22, 23 ; 121, 122, 123) présentent chacun une paroi dont l'épaisseur de paroi est respectivement uniforme ou non uniforme sur les sections d'un segment de ballonnet individuel (21, 22, 23 ; 121, 122, 123).

7. Cathéter (10 ; 100) selon l'une quelconque des revendications précédentes, **caractérisé en ce que** les segments de ballonnet (21, 22, 23 ; 121, 122, 123) présentent chacun une paroi ayant une épaisseur de paroi, les épaisseurs de paroi des segments de ballonnet (21, 22, 23 ; 121, 122, 123) étant différentes ou uniformes.

8. Cathéter (10 ; 100) selon l'une quelconque des revendications précédentes, **caractérisé en ce que** la surface extérieure des segments de ballonnet (21, 22, 23 ; 121, 122, 123) est revêtue d'une substance choisie parmi : un agent actif pharmaceutique, un revêtement hydrophile, un revêtement d'héparine ou une combinaison de ceux-ci.

9. Cathéter (10 ; 100) selon l'une quelconque des revendications précédentes, **caractérisé en ce qu'**il est prévu dans la lumière de fluide (15) un élément d'étanchéité (30) qui commande les segments de ballonnet individuels (21, 22, 23 ; 121, 122, 123).

10. Cathéter (10 ; 100) selon la revendication 9, **caractérisé en ce que** l'élément d'étanchéité (30) est réalisé de manière à pouvoir être commandé latéralement dans la lumière de fluide (15).

11. Cathéter (10 ; 100) selon l'une quelconque des revendications précédentes, **caractérisé en ce qu'**il présente en outre une prothèse endoluminale expansible chargée sur celui-ci.
